# EUROPEAN PATENT APPLICATION

(11) **EP 0 640 621 A2**
(43) Date of publication of application: **01.03.1995**
(21) Application number: 94306112.7
(22) Date of filing: 18.08.1994
(51) Int. Cl.: C07K 16/36, C12N 5/12, G01N 33/577

(54) **Anti-thrombin monoclonal antibody**

(30) Priority: 24.08.1993 JP 229652/93
(71) Applicant: ORIENTAL YEAST CO., LTD., Tokyo 174 (JP)
(72) Inventor: Nishino, Masato, 17-47, Shimoshinjo 5-chome, Osaka-shi, Osaka-fu (JP); Yamagata, Yoshiki, Suita-shi, Osaka-fu (JP); Fujita, Tsuyoshi, Ikeda-shi, Osaka-fu (JP)
(74) Representative: Ede, Eric

(57) **Abstract**

A novel anti-thrombin monoclonal antibody is prepared by using thrombin-antithrombin III complex (TAT) as an antigen; by using the monoclonal antibody, rapid, exact and simple immunoassay for not only TAT but also thrombin itself is possible.

## Description

### Field of the Invention:

The present invention relates to an anti-thrombin monoclonal antibody, which is useful as a reagent for immunoassay, for example, for quantitative determination of thrombin-antithrombin III complex or thrombin in plasma or serum.

### Prior Art:

Thrombin plays an important role in the system of blood coagulation, and its measurement is considered important in diagnosis and remedy of various diseases. To measure components in living organisms, immunoassay is employed in many cases.
In order to attain exact measurement of the component, preparation of an anti-thrombin monoclonal antibody is indispensable.

However, since thrombin has a weak immunogenicity and is unstable, it is impossible to prepare the monoclonal antibody, using it as an antigen. Nobody has succeeded in immunoassay for thrombin and thrombin-anti-thrombin III complex using the monoclonal antiboby, though desired by many people in this technical field.

The present invention has been attained on such a technical level. As described above, a hybridoma capable of producing an anti-thrombin monoclonal antibody that may be used in immunoassay for thrombin-antithrombin III complex and for thrombin has heretofore been unknown, and the present invention is the first that has obtained the hybridoma.

### Problems to be Solved by the Invention:

A small amount of circulating prothrombin is converted into thrombin after the start and the activation of the blood coagulation mechanism, in which, however, thrombin immediately forms a 1/1 (by mol) complex with antithrombin III (hereinafter referred to as ATIII).

It is presumed that some behavior of coagulation of circulating blood may be known by measuring the amount of the thrombin-antithrombin complex (hereinafter referred to as TAT) in blood. Therefore, it is possible to clarify the condition of the disease of a patient from the side of blood coagulation and to foresee the formation of thrombus or the disseminated intravascular coagulation (DIC) in its early stages.

In ordinary immunoassay kits for TAT, an thrombin specific polyclonal antibody is applied to the antibody fixed to a solid phase so as to evade the cross reaction with prothrombin in plasma. However, the polyclonal antibody used therein have various problems in that the specificity and the reactivity of the antibody are not always constant, the necessary amount of the antibody cannot always be ensured, and the absorbing operation is necessary so as to evade the cross reaction with prothrombin. We, the present inventors have attempted to produce a monoclonal antibody capable of specifically recognizing thrombin.

However, thrombin has a weak immunogenicity and is, of itself, a strong protease to decompose proteins in the blood of an immunized animal or to cause autolysis. Thus, thrombin is an extremely unstable substance and therefore it is problematic to use thrombin as an immunogen. For these reasons, it is impossible to efficiently produce a monoclonal antibody, using thrombin.

### Brief Explanation of Drawings:

Fig. 1 shows a TAT standard curve.

Fig. 2 shows a thrombin standard curve.

### Means for Solving the Problems:

Given the situations, we, the present inventors have converted our ideas and have used TAT obtained by reacting thrombin and ATIII, but not thrombin itself, as the immunogen. As a result, surprisingly and unexpectedly, we have obtained an extremely useful and novel knowledge that an anti-thrombin monoclonal antibody is obtained by immunization with TAT and that immunoassay for not only TAT but also thrombin is possible when the thus-obtained monoclonal antibody is used.

The present invention has been completed on the basis of these novel findings and after further studies of them. The present invention will be explained in detail hereunder.

To produce an anti-thrombin monoclonal antibody according to the present invention, any per-se well known technique for producing monoclonal antibodies may be employed. The most important characteristic of the present invention is to selectively use TAT as the antigen. The present invention, using TAT as the antigen, is the first that has succeeded in the production of an anti-thrombin monoclonal antibody. Therefore, the anti-thrombin monoclonal antibody itself is novel. The hybridoma to produce it is also novel. Using the monoclonal antibody, rapid, exact and simple immunoassay for not only TAT but also thrombin has become possible for the first time. The present invention will be explained more concretely hereunder.

### A. Preparation of Antigen:

Prothrombin purified from plasma is activated with Echis carinatus venom or with a mixture of Factor Va, Factor Xa and a phospholipid to be converted into thrombin, which is then reacted with ATIII purified from plasma to give TAT.

TAT is purified by ion exchange chromatography on a CM Sepharose CL-6B column (Biochemical et Biophysica Acta, 952 (1988), 37-47) and an affinity chromatography on an anti-ATIII antibody fixed column.

Alternatively, it is also possible to purify TAT from serum and plasma, using an anti-thrombin antibody fixed column, and anti-TAT neoantigen antibody fixed column, etc.

### B. Immunization of Mice with TAT:

Male BALB/c mice are generally used, but mice of other lines may also be used.

Mice are immunized repeatedly with TAT via an intraperitoneal, subepidamal or intravenous injection, until the antibody titer of serum rise satisfactorily. Several days after the final immunization, the spleen is taken out from each of the immunized mice under sterile conditions to be used for cell fusion.

### C. Formation of Hybridoma:

The spleen is taken out from each of the immunized mice under sterile conditions, the spleen cells are release therefrom and are fused with mouse myeloma cells in the presence of a suitable cell-fusion promoter. As myeloma cells to be used for the cell fusion, for example, mentioned are P3-Ag8-γ, P3-X63-Ag8, P3-X63-Ag8-Ul, NSI-Ag4/1, X63-Ag8-6.5.3, SP2/0-Agl4, MPC11-45, 6TGl.7, S194/5XX0.BU.l, etc.

As the preferred cell fusion promoter, for example, mentioned is polyethylene glycol having a mean molecular weight of from 1000 to 6000.

Electric pulse may also be used for the cell fusion.

### D. Screening of Hybridoma:

Since the myeloma cells to be used for the cell fusion are of a 8-azaguanine resistant strain and do not have hypoxanthine guanine phosphoribosyltransferase, they do not produce nucleotides in HAT media (containing hypoxanthine, aminoputerine and thymidine) and cannot live therein.

Therefore, after the cell fusion, the cells are incubated in a HAT medium for 10 to 14 days whereby only hybridomas formed by cell fusion of the spleen cells and the myeloma cells are screened.

### E. Establishment of Anti-thrombin Monoclonal Antibody-producing Hybridoma:

After the cell fusion, the cells are incubated in a HAT medium for 10 to 14 days, and the supernatant separated from the culture medium is collected and subjected to ELISA by which its reactivities with pure thrombin, TAT, ATIII and prothrombin are determined. From the results, the cells in the wells that have been proved to have a reactivity with thrombin are incubated in an HT medium (containing hypoxanthine and thymidine) to be scaled up, and these are cloned by a limiting dilution-culture method using non-immunized mouse spleen cells as the feeder cells.

After the cloning, the cells are again screened by ELISA, by which stable and well-growing clones that have high antibody producibility are selected as a anti-thrombin specific antibody producing hybridomas. After scaled up, the hybridomas are frozen for storage.

### F. Method for Producing Monoclonal Antibody:

To produce the intended monoclonal antibody, are employable an in-vitro method where the hybridomas are incubated in a serum-added medium or a serum-free medium and an in-vivo method where the hybridomas are implanted mouse intraperitoneally and the ascites is collected therefrom. The crude monoclonal antibody thus obtained may be purified by salting-out with ammonium sulfate, ion exchange column chromatography, molecular sieve column chromatography, protein A column chromatography, antigen fixed column chromatography, etc.

### G. Immunoassay for TAT and Thrombin, using Anti-thrombin Monoclonal Antibody:

Immunoassay includes, for example, turbidimetric immunoassay, latex fixation test, one-step sandwich EIA, two-step sandwich EIA, one-step sandwich RIA, two-step sandwich RIA, etc.

As the labeling substances to produce labeled antibodies, for example, usable are enzymes, fluorescent substances, luminescent substances, radioactive substances, etc. Enzymes include peroxidase, glucose oxidase, alkaline phosphatase, β-D-galactosidase, luciferase, etc.; fluorescent substances include fluorescein isothiocyanate, tetramethylrhodamine isothiocyanate, etc.; luminescent substances include luminol, acridinium esters, luciferin, etc.; and radioactive substances include ¹²⁵I, ¹³¹I, ¹⁴C, ³H, etc. However, these are not limitative, and any others that are usable in immunoassay may also be used.

Of these labeling substances mentioned above, preferred are enzymes. Binding of these labeling substances to the whole antibody, F(ab')² or Fab' may be formed by various methods such as a glutaraldehyde method, a periodic acid method or a maleimide method, etc.

As insoluble carriers to be used for fixing the antibody, for example, mentioned are polymers such as polystyrenes, polyethylenes, polypropylenes, polyesters, polyacryl nitriles, fluorine resins, crosslinked dextrans, polysaccharides, etc.; paper, glass, metal, agarose; and combinations of them.

The insoluble carriers may have various shapes, including, for example, tray-shaped, spherical, fibrous, rod-like, disc-like, container-like, cellular and tubular carriers.

### Examples:

The present invention will be explained in more detail by means of the following examples.

### Example 1:

### Preparation of TAT:

58.95 mg (3.93 mg/ml; 15 ml) of ATIII (Neuart® made by Green Cross Corp.) and 37,500 units (2,500 units/ml; 15 ml) of thrombin (Thrombin-Green Cross, made by Green Cross Corp.) were mixed in 20 mM Tris-HCl buffer (pH 7.4) containing 0.15 M NaCl, in a mole ratio of 2:1, and reacted at 37°C for 15 minutes to form TAT.

The reaction mixture was charged into a CM Sepharose CL-6B column (column capacity: 400 ml) that had been equilibrated with 0.01 M phosphate buffer (pH 6.4).

The column was washed with 2 liters of the above described buffer and then developed with a linear NaCl gradient to a limit concentration of 0.3 M (4 liters in total).

The TAT concentration, the thrombin activity and the ATIII concentration of each eluted fraction were measured, using commercial kits, and the fractions containing only TAT were collected.

The above described operation was repeated twice. Next, the collected fractions were charged into an affinity purified goat anti-ATIII polyclonal antibody fixed formylcellulofine column (column capacity: 14 ml) that had been equilibrated with 0.1 M Tris-HCl buffer (pH 7.5) containing 0.15 M NaCl and 0.1 % NaN3. The column was washed with 70 ml of the same buffer with the equilibration, from which TAT was eluted with 140 ml of 0.3 M potassium chloride-hydrochloric acid buffer (pH 2.3). The eluate was subjected to buffer exchange for 0.01 M phosphate buffer (pH 7.2) containing 1 mM benzamidine hydrochloride, 0.1 % NaN₃ and 0.15 M NaCl and then concentrated by ultrafiltration to obtain about 5 mg of pure TAT.

The obtained TAT was stored at -80°C and was used as an immunogen or an antigen for ELISA and sandwich EIA.

### Example 2:

### Immunization with TAT Antigen and Formation of Hybridoma:

### (a) Immunization with TAT Antigen:

50 µg of the above-mentioned TAT were intraperitoneallyadministered to a mouse in the form of its emulsion (500 µl) with a Freund's complete adjuvant. This is primary immunization.

After 2 weeks, the same amount of the TAT was subcutaneously administered to the same mouse in the form of its emulsion with a Freund's incomplete adjuvant. This is secondary immunization.

After about one month further but several days before the cell fusion to be effected layer, the same amount of the TAT in Dalbecco's phosphate buffered saline was intraperitoneally administered to the same mouse once or twice. This is the final immunization.

### (b) Cell Fusion:

The spleen was taken out from the TAT-immunized mouse under sterile conditions and cut into pieces with operating scissors in a modified RPMI 1640 medium (prepared by adding 140 mg/liter of L-glutamine, 55 mg/liter of sodium pyruvate, 10 mg/liter of L-cysteine, 3 mg/liter of ascorbic acid, 2 mg/liter of choline chloride, 3 mg/liter of glutathione, 50 mg/liter of streptomycin sulfate and 200,000 units/liter of penicillin G potassium to the RPMI 1640) containing 15% fetal calf serum, whereby the spleen cells were eluted out in the medium. The resulting spleen cell suspension was passed through a Nylon mesh and then centrifuged at 1,000 rpm for 5 minutes to collect the spleen cells, which were washed twice with the medium by centrifugation. The washed cells were again suspended in the medium, and the number of the living cells therein was counted.

15 × 10⁷ of the above described spleen cells were added to 3 × 10⁷ of pre-cultivated mouse myeloma cells of X63-Ag8-6.5.3 and well mixed in the modified RPMI 1640 medium. Then, these were washed twice by centrifugation.

Next, the supernatant was taken out by suction, one ml of 50 % polyethylene glycol 4000 solution that had been heated at 37°C was gradually added thereto while stirring over a period of one minutes, and the whole was gently stirred for further one minute. Next, 10 ml of the modified RPMI 1640 medium was gradually added thereto over a period of 5 minutes while still stirring, and this was centrifuged at 1000 rpm for 5 minutes to collect the fused cells.

The cells were suspended in 50 ml of an HT medium (prepared by adding 1 × 10⁻⁴ M hypoxanthine and 1.6 × 10⁻⁵ M thymidine to the modified RPMI 1640 medium) containing 15 % fetal calf serum.

To a 48-well plate, on which 1 x 10⁶ feeder cells (non-immunized mouse spleen cells) per 0.5 ml-well had been incubated at 37°C in an atmosphere containing 3 % carbon dioxide gas on the previous day, added was the suspension of the above-mentioned fused cells in an amount of 0.5 ml per well and incubated thereon at 37°C in the same atmosphere containing 3 % carbon dioxide gas.

After 18 hours, a half of the culture medium was exchanged for a HAT medium (prepared by adding 1.63 × 10⁻⁷ M aminoputerine to an HT medium) and thereafter a half of the culture medium was exchanged for the new HAT medium at desired intervals, whereupon the hybridomas growing in the HAT medium were screened.

### (c) Establishment of Anti-thrombin Monoclonal Antibody-producing Hybridoma:

The fused cells were incubated for 10 to 14 days, and the supernatant was collected from the culture medium. This was subjected to ELISA, by which its reactivities with pure thrombin (made by Enzyme Research Co.), TAT and ATIII ( made by Green Cross Corp.) and prothrombin (made by Enzyme Research Co.) were determined.

Various antigens (1 µg/ml) were added to Nunc-Immunoplate Maxisorp F96 (made by Nunc Co.) each in an amount of 100 µl/well and allowed to stand overnight at 4°C.

Next, the plate was washed three times each with phosphate buffer (pH 7.2) containing 0.1 % Tween 20 and 0.15 M NaCl and then blocked with phosphate buffer (pH 7.2) containing 0.1 % Bovine serum albumin and 0.15 M NaCl for 2 hours at room temperature.

After washed three times each with the above described buffer for washing, 100 µl of the previously-prepared culture supernatant were added to each well and reacted at room temperature for 2 hours.

After washed six times each with the above described buffer for washing, peroxidase-labeled rabbit anti-mouse IgG (H+L) antibody (made by Seikagaku KK) that had been diluted to a 1/1000 dilution with the same buffer was added to the plate in an amount of 100 µl per well and reacted for one hour at room temperature. This was then washed six times each with the above described buffer for washing. Then, a substrate solution (0.1 M phosphate buffer with pH of 6.0, containing 1.04 mg/ml of orthophenylenediamine, 0.009 % hydrogen peroxide and 0.15 M NaCl) was added to the plate in an amount of 100 µl per well and reacted for 7 minutes at room temperature. 4 N HCl was added thereto in an amount of 100 µl per well, by which the reaction was stopped. Using a colorimeter for microwell plate, the absorbance at 492 nm was measured.

On the basis of the measurement, the cells in the wells that had been admitted to have reacted with thrombin were selected and incubated in an HT medium containing 15 % fetal calf serum to be scaled up. Then, these were cloned by means of a limiting dilution method using non-immunized mouse spleen cells.

The cloned cells were again screened by the above-mentioned ELISA method to select the stable and well-growing clones having high antibody-producibility. Accordingly, antithrombin specific antibody producing hybridomas HT-1, HT-2, HT-3 and HT-4 were obtained. After having been scaled up, the cells were frozen for storage.

### Example 3:

### Production of Monoclonal Antibody:

### In-vitro Method:

It is possible to incubate these hybridomas in the modified RPMI medium containing 15 % fetal calf serum and to obtain the monoclonal antibody from the culture medium. In order to evade the contamins such as IgG, TAT, ATIII and prothrombin in fetal calf serum, a serum-free medium of Hybridoma SFM (made by Gibco Co.) was used in this example.

The hybridomas of each clone were incubated separately in the serum-free medium at 37°C in 5 % CO₂ for one week, and the culture medium was centrifuged at 3000 rpm for 20 minutes to obtain a supernatant therefrom. To this was gradually added ammonium sulfate while stirring, the final concentration of the ammonium sulfate added being 50 % saturation. After the addition, this was allowed to stand at 4°C for one hour and then centrifuged at 12000 rpm for 20 minutes to separate the precipitates therefrom.

The separated precipitates were dissolved in 10 mM phosphate buffer (pH 7.2) containing 0.15 M NaCl and then dialyzed against the same buffer of 500 times the volume of the solution.

The dialyzate was mixed with the same amount of a binding buffer (1.5 M glycine buffer containing 3 M NaCl; pH 9.0) and charged into a protein A agarose column that had been equilibrated with the binding buffer. After the column was washed with the same buffer of 10 times of the column volume, this was eluted with an eluting buffer (0.1 M citrate buffer; pH 3.0) of 5 times the volume of the same, and then the elute was exchanged buffer for 10 mM phosphate buffer (pH 7.2) containing 0.15 M NaCl and 0.1 % NaN₃. Thus, a pure monoclonal antibody was obtained.

### In-vivo Method:

Pristane (2,6,10,14-tetramethylpentadecane) was intraabdominally administered to retired BALB/c mice in an amount of 0.5 ml per mouse. On 14 days, the hybridomas of each clone that had been proliferated in vitro were implanted in an amount of 1 × 10⁷ cells per mouse.

After 10 to 14 days, about 3 to 10 ml of ascites were obtained from one mouse for each clone of the hybridomas, and the concentration of the antibody in the ascites was from 1 to 20 mg/ml.

The purification of the monoclonal antibody from the ascites was effected in the same manner as that for the above-mentioned in-vitro purification method.

### Example 4:

### Typing of Monoclonal Antibody:

Using Mouse Typer (Bio-Rad Co.), the monoclonal antibody derived from the supernatant of the culture medium containing the hybridomas of each clone was identified its class and sub-class of the mouse immunoglobulin.

The results obtained are shown in Table 1 below.

**Table 1**

| Monoclonal Antibody | Class, Sub-class of Immunoglobulin |
|---|---|
| HT-1 | IgG_{2b} |
| HT-2 | IgG_{2b} |
| HT-3 | IgG1 |
| HT-4 | IgG1 |

### Example 5:

### Test for the Specificity of Monoclonal Antibody:

The monoclonal antibody purified from the ascites in Example 3 was tested to identify its specificity, by ELISA.

Precisely, a pure antigen (thrombin, TAT, ATIII or prothrombin, each 1 µg/ml) was put into the wells of Nunc-Immunoplate Maxisorp F96 (made by Nunc Co.) in an amount of 100 µl/well and allowed to stand at 4°C overnight.

The wells were washed three times each with phosphate buffer (pH 7.2) containing 0.1 % Tween 20 and 0.15 M NaCl and then blocked with phosphate buffer (pH 7.2) containing 0.1 % calf serum albumin and 0.15 M NaCl for 2 hours at room temperature.

After the wells were washed three times each with the above described buffer for washing, 100 µl of the pure monoclonal antibody that has been stepwise diluted with the blocking buffer were added to each well and reacted at 37°C for 2 hours.

After washed six times each with the above described buffer for washing, peroxidase-labeled rabbit anti-mouse IgG (H+L) antibody (made by Seikagaku KK) that had been diluted to a 1/1000 dilution with the same buffer was added to each well in an amount of 100 µl per well and reacted for one hour at 37°C. The wells were then washed six times each with the above described buffer for washing. Then, a substrate solution (0.1 M phosphate buffer with pH of 6.0, containing 1.04 mg/ml of ortho-phenylenediamine, 0.009 % hydrogen peroxide and 0.15 M NaCl) was added to each well in an amount of 100 µl per well and reacted for 20 minutes at room temperature. 4 N HCl was added thereto in an amount of 100 µl per well, by which the reaction was stopped. Using a colorimeter for microwell plate, the absorbance at 492 nm was measured.

The results obtained are shown in Table 2 below.

**Table 2**

| | Thrombin | TAT | ATIII | Prothrombin |
|---|---|---|---|---|
| HT-1 | ++ | + | - | - |
| HT-2 | ++ | + | - | - |
| HT-3 | ++ | - | - | - |
| HT-4 | ++ | - | - | - |

### Example 6:

### Measurement of TAT Concentration by Sandwich EIA:

A pure TAT was added to human plasma and the TAT concentration in the resulting mixture was measured, using Enzygnost TAT (made by Behringwerke AG). This was used as a standard sample in the following test.

A pure anti-thrombin antibody (TH-1, 10 µg/ml) was put into the wells of Nunc-Immunoplate Maxisorp F96 (made by Nunc Co.) in an amount of 100 µl/well and allowed to stand at 4°C overnight.

The wells were washed three times each with phosphate buffer (pH 7.2) containing 0.1 % Tween 20 and 0.15 M NaCl and then blocked with phosphate buffer (pH 7.2) containing 0.1 % calf serum albumin and 0.15 M NaCl for 2 hours at room temperature.

After the wells were washed three times each with the above described buffer for washing, 100 µl of the previously-prepared sample were added to each well and reacted at 37°C for 2 hours.

After washed six times each with the above described buffer for washing, peroxidase-labeled rabbit anti-ATIII antibody (made by Daco Product Inc.) that had been diluted to a 1/1000 dilution with the same buffer was added to each well in an amount of 100 µl per well and reacted for one hour at 37°C. The wells were then washed six times each with the above described buffer for washing. Then, a substrate solution (0.1 M phosphate buffer with pH of 6.0, containing 1.04 mg/ml of ortho-phenylenediamine, 0.009 % aqueous hydrogen peroxide and 0.15 M NaCl) was added to each well in an amount of 100 µl per well and reacted for 30 minutes at room temperature. 4 N HCl was added thereto in an amount of 100 µl per well, by which the reaction was stopped. Using a colorimeter for microwell plate, the absorbance at 492 nm was measured.

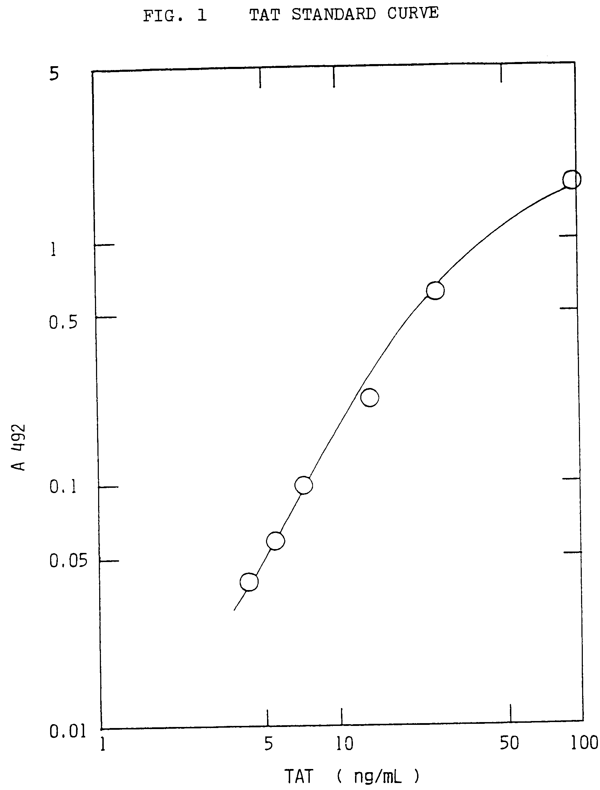

The results are shown in Fig. 1.

Both ATIII and prothrombin of up to 300 µg/ml each showed substantially no cross-reactivity with the antibody.

### Example 7:

### Measurement of Thrombin Concentration by Sandwich EIA:

A pure thrombin (made by Enzyme Research Co.) was diluted with phosphate buffer (pH 7.2) containing 0.1 % calf serum albumin and 0.15 M NaCl, and this was used as a standard sample in the following test.

Enzygnost TAT (made by Behringwerke AG) having rabbit antithrombin antibody-fixed tubes was used. 200 µl of the sample were added to each tube and reacted for 2 hours at 37°C. After the tubes were washed five times each with phosphate buffer (pH 7.2) containing 0.1 % Tween 20 and 0.15 M NaCl, glucose oxidase-labeled antithrombin monoclonal antibody (TH-2, 1 U/ml) was added to each tube in an amount of 100 µl per tube and reacted for 2 hours at 37°C. These were then washed six times each with the above described buffer for washing. Then, a substrate solution (0.1 M phosphate buffer with pH of 7.0, containing 0.055 mg/ml of ortho-phenylenediamine, 16.7 mg/ml of glucose and 33 µg/ml of peroxidase) was added to each tube in an amount of 200 µl per tube and reacted for 2 hours at room temperature. 4 N HCl was added thereto in an amount of 200 µl per tube, by which the reaction was stopped. The absorbance at 492 nm was measured.

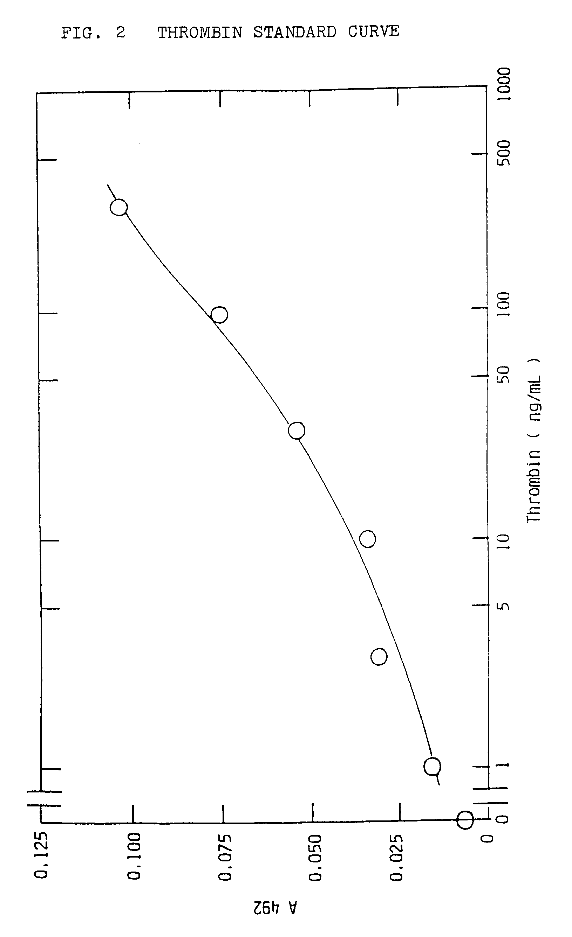

The results are shown in Fig. 2.

### Advantage of the Invention:

The present invention has employed TAT as the immunogen for the first time, resulting in the success in the efficient production of the anti-thrombin monoclonal antibody. The antibody is a novel substance. Using this, various immunoassay for quantitative and qualitative determination of not only TAT but also thrombin itself has become possible.

## Claims

1. An anti-thrombin monoclonal antibody producing hybridoma clone, obtained by fusing of spleen cells, immunized with thrombin-antithrombin III complex, and myeloma cells.

2. A monoclonal antibody reacting specifically to thrombin and thrombin-antithrombin III complex but not reacting to prothrombin and antithrombin III.

3. A monoclonal antibody reacting specifically to thrombin but not reacting to thrombin-antithrombin III complex, prothrombin and antithrombin III.

4. A method of immunoassay for thrombin-antithrombin III complex, composed of one or more anti-thrombin monoclonal antibodies as claimed in claim 2 or 3.

5. A method of immunoassay for thrombin, composed of one or more anti-thrombin monoclonal antibodies as claimed in claim 2 or 3.
